# EUROPEAN PATENT APPLICATION

(11) **EP 1 571 563 A2**
(43) Date of publication of application: **07.09.2005**
(21) Application number: 05002543.6
(22) Date of filing: 07.02.2005
(51) Int. Cl.: G06F 17/30

(54) **Sequence indexing method and system**

(30) Priority: 17.02.2004 KR 2004010400
(71) Applicant: SAMSUNG ELECTRONICS CO., LTD., Gyeonggi-do (KR)
(72) Inventor: Kim, Ki-eun, Seoul (KR); Hwang, Jung-joo, Yeongtong-gu Suwon-si Gyeonggi-do (KR)
(74) Representative: Grünecker, Kinkeldey, Stockmair & Schwanhäusser Anwaltssozietät

(57) **Abstract**

A sequence indexing method includes: parsing a patent document having a sequence number and a sequence list on the basis of a typical expression of a country to which the patent document was registered; first extracting the sequence number and the sequence list from the parsed patent document; and second extracting a sequence, which corresponds to the extracted sequence number, from the extracted sequence list.

## Description

### BACKGROUND OF THE INVENTION

### 1. Field of the Invention

The present invention relates to a sequence indexing method and system, and more particularly, to a method and system of indexing a sequence corresponding to a sequence number included in a patent document.

### 2. Description of the Related Art

As the Internet is developed, several methods are known to search a database for data or a document by using a key word. In order to obtain better search results, a study has been vigorously made to more easily and accurately process desired contents by embodying a natural language processing system.

However, it is difficult to search the document for an encrypted sequence, such as gene information in a conventional method. Since the gene sequence is encrypted using the alphabet characterized by repetition, and comprised of a character string of at least ten to more than one hundred thousand to the maximum, several algorithms are under development regarding a search method of a gene sequence. As well known methods, there are BLAST, FASTA and the like. These methods compare an amount of similarity between a known gene sequence and other comparative gene sequences registered to the database.

As the Human Genome project is completed and biotechnology is rapidly developed, great added values is generated while researchers work to analyze and understand the functions of genes. In order to protect the genes as the right, all countries claim the gene sequence as a patent right.

As the gene sequence is claimed as a patent, a sequence description is unified in a specification to rapidly and accurately examine the sequence, mainly in World Intellectual Property Organization (WIPO), United States, Japan, Europe and the like.

However, if the sequence is described in a document, the document has a page number of several tens to several hundreds due to a characteristic of the gene sequence. Accordingly, when patent information related with the gene sequence is collected, a long time is needed to analyze a number of patents. Therefore, an automatic sorting method is needed. However, as aforementioned, a conventional search method needs a long time and has a difficulty in obtaining valuable results when a gene sequence is searched in a patent document database.

Accordingly, when a gene sequence described in the patent is searched and the patent right is analyzed, a process of extracting the gene sequence is of great importance. However, it is difficult to automatize non-sorted documents and there is a high possibility of error in the automatic sorting process. Specifically, a conventional automatic document indexing method, such as an inverted file, a suffix array and a signature file has a drawback in that its embodiment is complicated and the exactness of operation is poor.

A conventional search method or similarity comparison method has a low efficiency and exactness in acquiring a wanted patent.

As an alternative, Patent Offices of countries around the world are attempting to index to search for a gene sequence. However, since the indexing work is still in an initial stage and is designed for easy application and examination, not for the patent search, a patent search is unsatisfactory.

### SUMMARY OF THE INVENTION

The present invention provides a sequence indexing method and system in which sequence information included in a registered or published patent document is quickly and accurately extracted and indexed in a typical expression method.

Also, the present invention provides a computer-readable recording medium for recording program that executes, in a computer, a sequence indexing method in which sequence information included in a registered or published patent document is quickly and accurately extracted and indexed in a typical expression method.

According to an aspect of the present invention, there is provided a sequence indexing method including: parsing a patent document having a sequence number and a sequence list on the basis of a typical expression of a country to which the patent document was registered; first extracting the sequence number and the sequence list from the parsed patent document; and second extracting a sequence, which corresponds to the extracted sequence number, from the extracted sequence list.

According to another aspect of the present invention, there is provided a sequence indexing method including: receiving a search query for a patent document search; searching and acquiring a patent document from a first database for storing at least one patent document, on the basis of the search query; first extracting a sequence list and a sequence number of the claim from the acquired patent document; and second extracting a sequence corresponding to the extracted sequence number from the extracted sequence list.

According to a further another aspect of the present invention, there is provided a sequence indexing system including: a document acquisition unit which acquires a patent document having a sequence number and a sequence list; a sequence list extraction unit which extracts the sequence list from the patent document on the basis of a typical expression of a country to which the patent document was registered; a sequence number extraction unit which extracts the sequence number from the patent document on the basis of the typical expression of the country to which the patent document was registered; and a sequence extraction unit which extracts a sequence corresponding to the extracted sequence number from the extracted sequence list.

### BRIEF DESCRIPTION OF THE DRAWINGS

The above and other features and advantages of the present invention will become more apparent by describing in detail exemplary embodiments thereof with reference to the attached drawings in which:
FIG. 1A is a flowchart illustrating a sequence indexing method according to one embodiment of the present invention;
FIG. 1 B is a flowchart illustrating a sequence indexing method according to another embodiment of the present invention;
FIG. 2 is a view illustrating a construction of a sequence indexing system according one embodiment of the present invention;
FIG. 3 is a view illustrating a construction of a sequence indexing system according another embodiment of the present invention;
FIG. 4 is a view illustrating a whole system employing a sequence indexing system according to the present invention;
FIG. 5 is a view illustrating an example of extracting a sequence number through an Internet connection to the U.S. Patent & Trademark Office;
FIG. 6 is a view illustrating an example of extracting a sequence list for a sequence number "SEQ ID NO:108" extracted in FIG. 5;
FIG. 7 is a view illustrating an example of storing a sequence list corresponding to a sequence number in database; and
FIG. 8 is a view illustrating an example of displaying sequence information, which is indexed through a sequence indexing system, for a user.

### DETAILED DESCRIPTION OF THE INVENTION

The attached drawings for illustrating preferred embodiments of the present invention are referred to in order to gain a sufficient understanding of the present invention, the merits thereof, and the objectives accomplished by the implementation of the present invention.

Hereinafter, the present invention will be described in detail by explaining preferred embodiments of the invention with reference to the attached drawings. Like reference numerals in the drawings denote like elements.

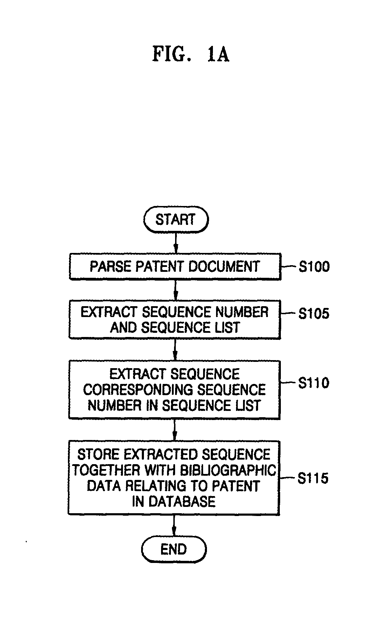

FIG. 1A is a flowchart illustrating a sequence indexing method according to one embodiment of the present invention.

Referring to FIG. 1A, a patent document is first parsed (S100). The patent document includes a specification, a claim, necessary drawings, necessary sequence lists, and bibliographic data related with the patent document. The sequence list attached to the patent document is prepared on the basis of a predetermined form, which is determined in each of the countries, or is prepared on the basis of a standard form of WIPO.

Here, the sequence list refers to a portion of the specification, which is attached to a patent application, or refers to a portion of a document, which is submitted after the filing of the application. The sequence list refers to a detailed disclosure of a gene sequence, that is, a sequence of a nucleic acid and amino acid sequence, and other available information. The sequence number is, as a sequence identifier, an integer given to each sequence of the sequence list.

Generally, the sequence of the sequence list is cited on the basis of the sequence identifier in the detailed description of the invention, the claims or the drawings, and is preceded by "SEQ ID NO:." A sequence citation method is similar, but can be different according to a patent preparation method in each country. In this case, the inventive sequence indexing method is embodied according to the preparation method of each country.

The patent document can be prepared using Hyper Text Markup Language (HTML), Standard Generalized Markup Language (SGML), and various document expression methods. For example, if the patent document is provided in an HTML format, the patent document is converted into a character string by removing HTML tags. A large-sized patent document is converted into at least two characters strings. Further, an unnecessary blank is removed from the patent document, thereby reducing a size of the character string.

Additionally, a parsing process is performed, as a word-oriented and sentence-oriented syntactic analysis, for the patent document converted into at least one character string.

After that, the sequence number and the sequence list, which are described in the typical form, are extracted from the converted patent document (S105). Generally, the sequence number is described, including "SEQ ID NO:", and the sequence list is attached to the specification as a separate paragraph. Further, the sequence number is described using the typical expression in the specification, the claims and the drawings. In case where it is intended to search for the sequence number related to the claims, that is, the sequence number related to the scope of a patent right, the sequence number included in the claims is extracted. The extracted sequence number and sequence list is stored in each character string variable.

The same sequence number can be repeatedly extracted in the patent document, and can be extracted even in the claims. Accordingly, it is inefficient that the same sequence is repeatedly extracted from the sequence list on the basis of the same extracted sequence number. Therefore, in order to increase the efficiency of the extraction, the sequence is extracted only once for the same sequence number.

The extracted sequence list is searched so that a sequence corresponding to the extracted sequence number (S110) can be extracted. In other words, each of the extracted sequence numbers is stored in each character string variable. Each of the sequences is extracted from the extracted sequence list on the basis of the sequence number stored in each character string variable. Or, the sequence can be extracted from the sequence list whenever the sequence number is extracted.

The extracted sequence is indexed and stored in the database together with the bibliographic data of the patent document (S115). The bibliographic data of the patent document includes a filing date, an applicant, a patentee, a patent period, a title of an invention, and the like. Therefore, after the sequence corresponding to the sequence number in the claims is extracted, the extracted sequence is indexed and stored in the database together with the bibliographic data and then, the sequence information, which the user desires, is searched for and provided from the database.

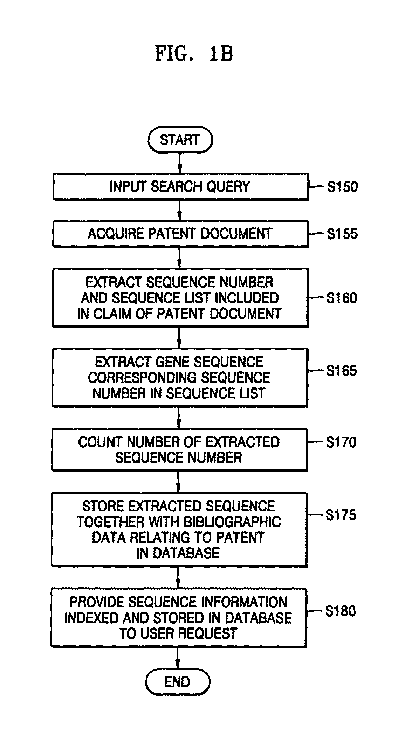

FIG. 1 B is a flowchart illustrating a sequence indexing method according to another embodiment of the present invention.

Referring to FIG. 1 B, a search query is inputted to search for the patent document (S150). The database for storing the patent document therein is searched on the basis of the inputted search query to acquire a corresponding patent document (S155). As the search query for acquiring the patent document, there are a patent number, a filing number, a patentee, an application publication number, a registration notification number or the like.

For example, in case where the patent document is searched using the patentee as the search query, a plurality of corresponding patent documents can be provided in the database. In this case, the plurality of patent documents is sequentially indexed.

The sequence list and the sequence number included in the claims are extracted from the acquired patent document (S160). Additionally, the sequence corresponding to the extracted sequence number is extracted from the extracted sequence list (S165). A process of extracting the sequence number, the sequence list and the sequence has been in detail described in FIG. 1A.

Since the claims include more than one sequence number, the sequence number extracted from the claims is counted in number (S170). At this time, the same sequence number is not again counted. Further, the sequence number included in the whole of the patent document can be counted in number.

The extracted sequence, the bibliographic data of the patent document, and the number of the sequence number are indexed and stored in the database together with the sequence number (S175). The sequence information indexed and stored in the database is provided according to a user's request (S180). The user can request the sequence information stored in the database, on the basis of the sequence number. Or, the user can request the sequence information stored in the database, on the basis of the patentee as the bibliographic data.

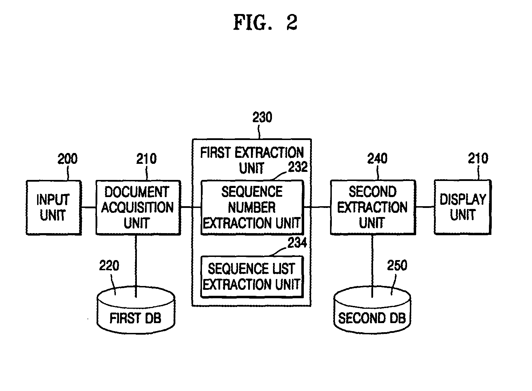

FIG. 2 is a view illustrating a construction of a sequence indexing system according one embodiment of the present invention.

Referring to FIG. 2, the sequence index system includes an input unit 200, a document acquisition unit 210, a first database 220, a first extraction unit 230, a second extraction unit 240, a second database 250, and a display unit 260. The first extraction unit is comprised of a sequence number extraction unit and a sequence list extraction unit.

The input unit 200 receives the search query from the user who intends to search for the sequence information of the patent document. The document acquisition unit 210 searches the first database 220 in which the patent document is stored, on the basis of the received search query to acquire a corresponding patent document. For example, if the input unit 200 receives any one of the patent number, the application number and the patentee as the search query, the document acquisition unit 210 searches the first database 220 on the basis of the patent number, the application number, or the patentee.

The first database 220 is provided to the Patent Office of each country, or is installed in a separate server to provide all patent documents. For example, in case where one intends to search for the patent document of the U.S. Patent & Trademark Office (USPTO) on the basis of the patent number, the user connects through Uniform Resource Locator (URL), which is the patent search site address of the USPTO, and then acquires the patent document corresponding to the patent number.

Further, the document acquisition unit 210 parses the patent document extracted from the first database unit 220. For example, in the case where the patent document is prepared using HTML, the patent document is converted into the character string after the HTML tags, the blanks between paragraphs, and the like are removed from the patent document. Further, in case where the patent document is prepared in a predetermined method and includes the tag and the like depending on the method, the patent document is converted into the character string after the tags are removed from the patent document.

The first extraction unit 230 extracts the sequence number and the sequence list from the patent document acquired by the document acquisition unit 210. For example, the first extraction unit 230 extracts the sequence number from the patent document converted into the character string, by using "SEQ ID NO:" As the expression method of the sequence number may vary with the country, the sequence number is extracted in a typical expression method of each country. The first extraction unit 230 stores the extracted sequence number and sequence list in each character string variable. In case where it is intended to index the sequence information related with the claims, the first extraction unit 230 extracts the sequence number stored in the claims.

The second extraction unit 240 extracts a corresponding sequence from the extracted sequence list on the basis of the extracted sequence number. In detail, the second extraction unit 240 searches the sequence list stored in the character string variable by the first extraction unit 230, on the basis of the sequence number stored in the character string variable, to extract the sequence corresponding to the sequence number. Additionally, the sequence number and the sequence corresponding to the sequence number are stored in the second database 250.

The display unit 260 displays the indexed sequence information together with the bibliographic data to the user. As the display unit 260, a printer, a monitor and the like can be used.

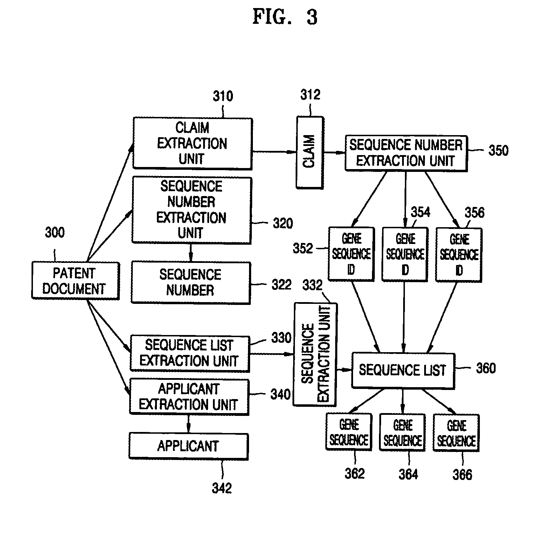

FIG. 3 is a view illustrating a construction of a sequence indexing system according another embodiment of the present invention.

Referring to FIG. 3, the sequence index system includes a claim extraction unit 310, a sequence-numbers extraction unit 320, a sequence list extraction unit 330, an applicant extraction unit 340, a sequence number extraction unit 350 and a sequence extraction unit 360.

The claim extraction unit 310 extracts the claim 312 from the patent document 300. The claim extraction unit 310 stores claims of the claim 312 in the character string variable. The sequence number extraction unit 350 extracts the sequence-numbers 352 to 356 from the extracted claim 312.

The sequence-numbers extraction unit 320 counts the sequence number included in the patent document 300 or the claims 312. Redundant sequence number are not counted.

The sequence list extraction unit 330 extracts the sequence list 332 included in the patent document. The applicant extraction unit 340 extracts the bibliographic data included in the patent document 300. The bibliographic data includes applicant information, patentee information, the patent number and the like.

The sequence extraction unit 360 searches the sequence list 332, which is extracted by the sequence list extraction unit 330, on the basis of the sequence numbers 352 to 356, which are extracted by the sequence number extraction unit 350, to extract corresponding sequences 362 to 366.

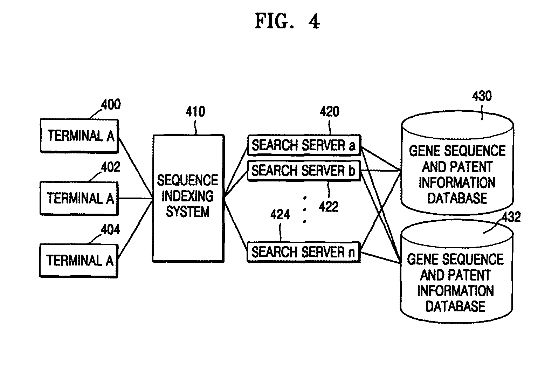

FIG. 4 is a view illustrating a whole system employing the sequence indexing system according to the present invention.

Referring to FIG. 4, the inventive sequence indexing system 410 is connected with at least one terminal 400 to 404 and at least one search server 420 to 424. The search server 420 to 424 is connected with at least one database 430 and 432.

If the user inputs the patent number, which is intended to search, through the terminals 400 to 402, the sequence indexing system 410 connects to any one of the search server 420 to 424 to request the patent document corresponding to the patent number. The search servers 420 to 424 search the databases 430 and 432 for the patent document corresponding to the patent number to extract the patent document and transmit the extracted patent document to the sequence indexing system 410.

Accordingly, the sequence indexing system 410 extracts and stores the sequence number, the sequence list, the number of the sequence number and the bibliographic data in the databases 430 and 432. The sequence information acquired by extracting and indexing the patent document, the sequence number and the like, which are not indexed, can be stored and managed in the same database or can be stored and managed in a separate database.

The sequence indexing system 410 according to one embodiment of the present invention is comprised of a DataBase Management System (DBMS) database table comprised of MySQL and program prepared using PERL. At this time, MySQL or Relation DataBase Management System (RDBMS) having a function corresponding to the MySQL, and ActivePERL are required as a previous preparation environment. Any one of PERL Interpreter Windows family Operating System (OS), UNIX family OS, and LINUX family OS is required. Further, in an Internet connectivity Intranet environment, a LAN connectivity RDMBS and the PERL can be used in a separate different system. Hereinafter, an example of embodying the sequence indexing system using the PERL is described on the basis of a U.S. patent document.

In case where the sequence indexing system is embodied using the PERL, the patent number is taken as an argument such that a full patent text is available to be stored in the character string variable. The claims are extracted through the typical expression from the character string for the full patent text. The full text of the extracted claims is returned. At this time, the typical expression used for the extraction of the claims is as follows:
" 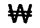 nClaims 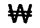 n((.| 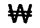 n)*) 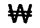 n Description 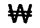 n".

Additionally, the number of the sequence number is extracted through the typical expression from the character string variable for the full patent text, and the number of the extracted sequence number is allocated to an integer variable. At this time, the typical expression used for the extraction of the number of the sequence number is as follows:
"NUMBER 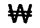 sOF 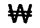 s(SEQ 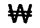 sID 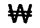 sNOS|SEQUENCES): 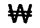 s(.*) 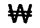 n".

In a next step, the sequence list is extracted through the typical expression from the character string variable for the full patent text, and the extracted sequence list is returned. At this time, the typical expression used for the extraction of the sequence list is as follows:
" 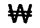 s+SEQUENCE LISTING 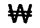 n)).| 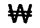 n)*".

The sequence numbers are extracted through the typical expression from the character string variable for the extracted claims. After that, a redundancy check and a redundancy removal of the extracted sequence numbers are performed, and then the extracted sequence numbers are stored in an array, and then returned. At this time, the typical expression used for the extraction of the sequence numbers is as follows:
"SEQ 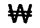 s+ID 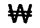 s+NO(: 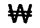 s|( 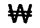 . 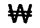 s)) 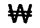 s*([0-9]+)"

After the sequence list and the sequence number are extracted, the sequence corresponding to the sequence number is extracted from the character string variable for the sequence list through the typical expression prepared using the sequence number stored in the array. Additionally, the extracted sequence is returned in a character string type.

In order to extract the bibliographic data from the patent document, applicant information is extracted through the typical expression from the character string variable for the patent document, and then the extracted applicant information is allocated to the character string variable. After that, the stored character string variable is returned. Necessary bibliographic data such as the patent document besides the applicant is extracted in the same manner. At this time, the typical expression for extracting the applicant information is as follows:
"Assignee: 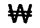 n( 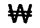 s*) 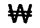 n(.*) 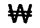 n"

As described above, the patent number, the applicant, the number of the sequence, the number of the sequence described in the claims, the sequence list described in the claims and the like are extracted and allocated to the variable. Through the connection to the database, each of the variables is inputted as a bind-parameter to the database through an SQL insert text.

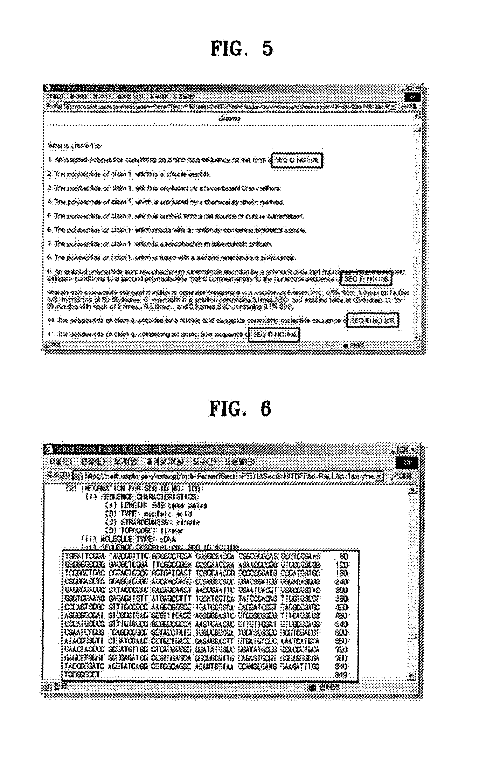

FIGS. 5 through 8 are views illustrating each of processes of the sequence indexing method according to an embodiment of the present invention.

FIG. 5 is a view illustrating an example of extracting the character string through an internet connection to U.S. Patent & Trademark Office. In FIG. 5, the claim is illustrated, and the sequence number is distinguished as "SEQ ID NO:" The claims include four sequence numbers, but include the same two sequence numbers. As a result, two sequence numbers can be extracted from the claims.

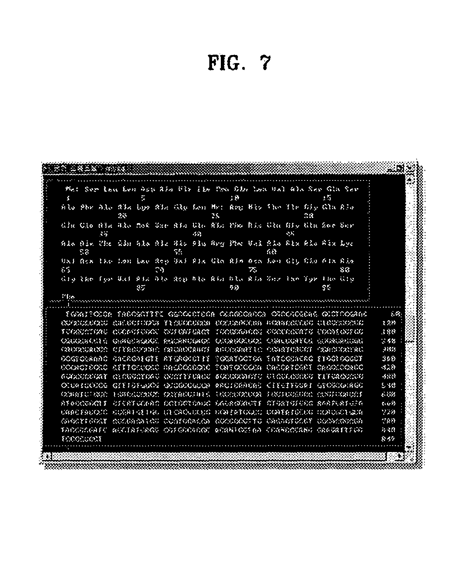

FIG. 6 illustrates an example of extracting the sequence list for the sequence number "SEQ ID NO:108" extracted in FIG. 5, and illustrates information on the extracted sequence number and the sequence list corresponding to the sequence number.

FIG. 7 is a view illustrating an example of storing the sequence list, which corresponds to the sequence number, in the database. The sequence list corresponding to the sequence number is indexed and stored in the database, and the corresponding sequence list is provided according to the user's request.

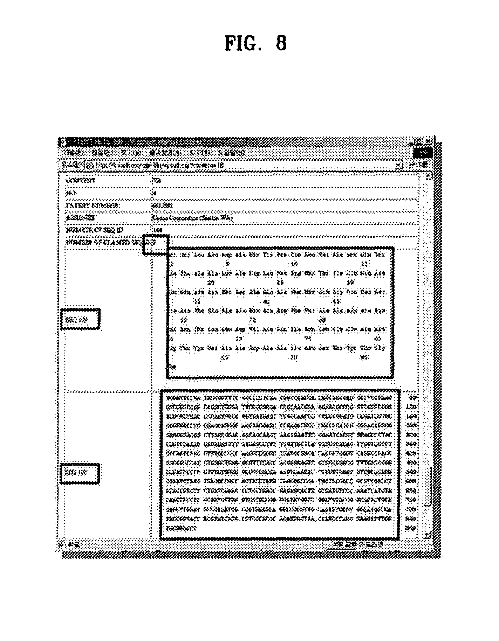

FIG. 8 is a view illustrating an example of displaying sequence information, which is indexed through the sequence indexing system, for the user. After the sequence number, the sequence list, the number of the sequence number and the bibliographic data are extracted from the patent document, the resulting data is displayed for the user. The sequence information is expressed and displayed together with information on a rightful person on a screen or paper and other display units for the user according to a predetermined output method. After that, it is stored in the database.

According to the present invention, the sequence range is concurrently recognized using the typical expression in a process of position analysis and tagging of the sequence within the patent document, thereby more accurately and quickly acquiring the sequence information through the extraction, storage and expression method of the sequence.

Further, in case where the searching and sorting work of the patent document depending on the sequence number is performed for the patent document received through the Internet, the accurate and quick search results are provided.

The inventive sequence indexing system, which functions as a sequence separator in the patent document based on the typical expression, can be associated with other databases including the gene sequence, and can be implemented for analysis in association of the general database including a gene sequence with the patented sequence information.

The sequence specified in the claims includes industrially available information distinguished from the general gene sequence, or includes information on the sequence known as a cause of disease or a special variation state. Accordingly, the present invention is beneficial to analysis of a sequence right to set a patent strategy for a related technology.

In comparison of a normal state with a variation state (for example, the cases where the sequence is inserted, deleted and substituted such as mutation) of a gene sequence, information on the functions of genes can be easily acquired and is helpful for the development of a diagnostic product or a medical treatment. Accordingly, the present invention extracts the gene sequence specified in the claims within a short time, and provides an understanding of the rightful person and the usage of the gene sequence. Therefore, the present invention is helpful for setting the patent strategy for the right of a similar sequence.

Further, the inventive sequence indexing method is useful when a sequence related to a disease or a sequence relating to specific functions is extracted and secured in a large amount within a short time, and the right thereof is analyzed to seek commercial use.

The invention can also be embodied as computer-readable codes on a computer-readable recording medium. The computer-readable recording medium is any data storage device that can store data which can be thereafter read by a computer system. Examples of the computer-readable recording medium include read-only memory (ROM), random-access memory (RAM), CD-ROMs, magnetic tapes, floppy disks, optical data storage devices, and carrier waves (such as data transmission through the Internet). The computer-readable recording medium can also be distributed over network-coupled computer systems so that the computer-readable code is stored and executed in a distributed fashion.

While the present invention has been particularly shown and described with reference to exemplary embodiments thereof, it will be understood by those of ordinary skill in the art that various changes in form and details may be made therein without departing from the spirit and scope of the present invention as defined by the following claims.

## Claims

1. A sequence indexing method comprising:
parsing a patent document having a sequence number and a sequence list on the basis of a typical expression of a country to which the patent document was registered;
first extracting the sequence number and the sequence list from the parsed patent document; and
second extracting a sequence, which corresponds to the extracted sequence number, from the extracted sequence list.

2. The sequence indexing method of claim 1, wherein the patent document is prepared according to a preparation standard of a sequence list of World Intellectual Property Organization (WIPO).

3. The sequence indexing method of claim 1, wherein the parsing of the patent document comprises: removing HTML (Hyper Text Markup Language) tags and a blank from the patent document prepared using HTML and then, converting the patent document into a character string.

4. The sequence indexing method of claim 1, wherein the first extracting comprises:
extracting claims from the patent document;
extracting the sequence number from the claims; and
extracting the sequence list from the patent document.

5. The sequence indexing method of claim 1, wherein the first extracting comprises: checking for any redundancy in the extracted sequence number in order to remove the redundant sequence number.

6. The sequence indexing method of claim 1, further comprising: counting the extracted sequence number in number.

7. The sequence indexing method of claim 6, wherein the counting of the number comprises: counting a sequence number, which is not redundant with the extracted sequence number.

8. The sequence indexing method of claim 1, further comprising: after bibliographic data is extracted from the patent document, storing a sequence corresponding to the bibliographic data and the extracted sequence number in a database.

9. The sequence indexing method of claim 8, wherein the bibliographic data comprises a patentee, the number of the extracted sequence number and a patent number.

10. The sequence indexing method of claim 1, further comprising:
displaying the bibliographic data and the sequence corresponding to the extracted sequence number.

11. A sequence indexing method comprising:
receiving a search query for a patent document search;
searching and acquiring a patent document from a first database for storing at least one patent document, on the basis of the search query;
first extracting a sequence list and a sequence number of the claim from the acquired patent document; and
second extracting a sequence corresponding to the extracted sequence number from the extracted sequence list.

12. The sequence indexing method of claim 11, wherein the acquiring of the patent document comprising: connecting to URL (Uniform Resource Locator) of a site at which the first database is located, to acquire a patent document corresponding to the search query.

13. The sequence indexing method of claim 11, further comprising: storing sequence information corresponding to the extracted sequence number, and bibliographic data of the patent document, in a second database.

14. The sequence indexing method of claim 13, wherein the acquiring of the patent document comprises: if sequence information of the patent document corresponding to the search query exists in the second database, providing sequence information stored in the second database.

15. The sequence indexing method of claim 11, wherein the acquiring of the patent document comprises: searching for the patent document by using any one of a patent number, an applicant, and a patentee as a search query.

16. A sequence indexing system comprising:
a document acquisition unit which acquires a patent document having a sequence number and a sequence list;
a sequence list extraction unit which extracts the sequence list from the patent document on the basis of a typical expression of a country to which the patent document was registered;
a sequence number extraction unit which extracts the sequence number from the patent document on the basis of the typical expression of the country to which the patent document was registered; and
a sequence extraction unit which extracts a sequence corresponding to the extracted sequence number from the extracted sequence list.

17. The sequence indexing system of claim 16, wherein the sequence number extraction unit extract the sequence number provided in the claims of the patent document.

18. The sequence indexing system of claim 16, wherein the document acquisition unit converts a HTML patent document, in which a HTML tag and a blank are removed from, into the character string.

19. The sequence indexing system of claim 16, further comprising a database which stores a gene sequence corresponding to the extracted sequence number, and bibliographic data of the patent document.

20. The sequence indexing system of claim 16, further comprising a display unit which displays a sequence corresponding to the extracted sequence number together with the bibliographic data.

21. The sequence indexing system of claim 16, further comprising an input unit which receives a search query for searching for the patent document,
wherein the document acquisition unit acquires the patent document from the first database for storing at least one patent document, on the basis of the search query.

22. The sequence indexing system of claim 16, wherein the document acquisition unit connects to URL at which the first database is located, to acquire a patent document corresponding to the search query.

23. The sequence indexing system of claim 16, further comprising a second database which stores sequence information, which corresponds to the extracted sequence number, and bibliographic data of the patent document.

24. The sequence indexing system of claim 23, wherein if sequence information of the patent document corresponding to the search query exists in the second database, the document acquisition unit provides sequence information stored in the second database.

25. A computer-readable recording medium which records program for executing a sequence indexing method of claim 1 or 11 in a computer.
